# EUROPÄISCHE PATENTSCHRIFT

(11) **EP 1 694 286 B1**
(45) Veröffentlichungstag und Bekanntmachung des Hinweises auf die Patenterteilung: **04.03.2009**
(21) Anmeldenummer: 04819660.4
(22) Anmeldetag: 01.12.2004
(51) Int. Cl.: A61Q 19/00, A61K 8/31, A61K 8/22, A61K 8/89

(54) **KOSMETISCHES UND DERMATOLOGOSCHES SAUERSTOFF-TRÄGERSYSTEM**
COSMETIC AND DERMATOLOGICAL OXYGEN CARRIER SYSTEM
SYSTEME DE SUPPORT COSMETIQUE ET DERMATOLOGIQUE POUR OXYGENE

(30) Priorität: 08.12.2003 DE 10358306
(43) Veröffentlichungstag der Anmeldung: 30.08.2006
(73) Patentinhaber: Coty B.V., 2031 CC Haarlem (NL)
(72) Erfinder: GOLZ-BERNER, Karin, MC-98000 (MC); ZASTROW, Leonhard, MC-98000 (MC); MOYON, Roselyne, F-06110 Le Cannet (FR)
(74) Vertreter: Gulde, Klaus W.
(86) Internationale Anmeldenummer: PCT/EP2004/013856
(87) Internationale Veröffentlichungsnummer: WO 2005/053636

(56) Entgegenhaltungen:
- EP-A- 0 670 159
- DE-A1- 4 333 561
- DE-A1- 19 907 305
- US-A- 4 569 784
- US-A- 5 219 844
- US-A- 5 637 318
- US-A- 5 874 093
- US-A- 5 885 564
- US-A1- 2003 103 922
- US-B1- 6 403 109
- US-B1- 6 419 909

## Beschreibung

Die Erfindung betrifft ein Sauerstoff-Trägersystem, das geeignet ist, gasförmigen Sauerstoff in kosmetischen und dermatologischen Anwendungsformen auf die Haut zu bringen.

Es ist bekannt, Perfluorcarbonverbindungen als Sauerstoffträger in kosmetischen Anwendungsformen einzusetzen. Die WO 94/0098 beschreibt asymmetrische lamellare Aggregate, bestehend aus Perfluorcarbonen und Phospholipiden mit einem Phosphatidylcholingehalt von wenigstens 30 Gew-%. In modernen kosmetischen Formeln werden zunehmend Siliconöle verarbeitet, um bestimmte vorteilhafte Eigenschaften, wie Leichtigkeit, keine Klebrigkeit, Reichhaltigkeit zu erzielen. In derartige Formeln lassen sich jedoch die bekannten Sauerstoff-Trägersysteme nur in geringen Konzentrationen von maximal 5 Gew-% einarbeiten, weil danach Stabilitätsprobleme bei den Formeln auftreten. Geringe Konzentrationen derartiger bekannter Sauerstoff-Transportsysteme bedingen entsprechend geringere Sauerstoffgehalte in der Formulierung und dementsprechend in der Wirksamkeit für die Haut.

Weiterhin nachteilig bei den bekannten Systemen ist es, dass in siliconölfreien Formulierungen relativ hohe Konzentrationen des Trägersystems von 10 bis 40 Gew-% erforderlich sind, um Sauerstoff-Abgabemengen über längere Zeiträume von 8 bis 40 Wochen zu gewährleisten.

Die DE 199 07 305 beschreibt im Beispiel 2 ein Produkt, das aus vier separaten, teilweise gefärbten Phasen besteht, wobei eine Phase Perfluorodecalin sein soll und die weiteren Siliconöl, Wasser und Fett sein sollen. Die US-B-64 03 109 beschreibt eine Parfümemulsion, die in Beispiel 1 neben Parfümöl Perfluorodecalin und ein Siliconöl sowie Wasser enthält mit einem unterschiedlichen Brechungsindex von kontinuierlicher und disperser Phase.

Aufgabe der Erfindung ist die Bereitstellung eines neuen Sauerstoff-Trägersystems, das in siliconölhaltigen kosmetischen oder dermatologischen Formulierungen ohne Stabilitätsprobleme für die Formulierung beliebig einsetzbar ist und dessen Wirksamkeit in bezug auf die Sauerstoffabgabe deutlich erhöht ist.

Erfindungsgemäß ist das kosmetische und dermatologische Sauerstoff-Trägersystem dadurch gekennzeichnet, dass es aus einem flüssigen perfluoriertem oder teilfluoriertem Kohlenwasserstoff oder Kohlenwasserstoffgemisch mit einem Anteil von 0,1-10 Gew-%, einem flüssigen Siliconpolymeren oder Siliconpolymerengemisch mit einem Anteil von 10-85 Gew-% und einer Öl- oder Wasserbasis mit einem Anteil von 5-25 Gel-% besteht, wobei alle Gewichtsanteile auf das Gesamtgewicht des Trägersystems bezogen sind.

In einer vorteilhaften Ausführungsform ist das Sauerstoff-Trägersystem mit einem Anfangs-Sauerstoffgehalt von 150-950 mbar O₂ beladen.

Vorzugsweise beträgt der Anfangs-Sauerstoffgehalt 250 bis 400 mbar O₂ bereits bei Anteilen von 2-5 Gew-% perfluoriertem Kohlenwasserstoff.

In einer weiteren vorteilhaften Ausführungsform ist das Sauerstoff-Trägersystem mit einem Sauerstoffgehalt vier Wochen nach der Beladung in Höhe von 5-40 Vol-% des Anfangs-Sauerstoffgehaltes beladen, vorzugsweise 15-25 Vol-%.

Die Sauerstoffbeladung eines erfindungsgemäßen Systems erfolgt im allgemeinen so, dass nach Herstellung des Transportsystems Sauerstoffgas mit einem Partialdruck von 180 bis 600 mbar durch die Gesamtzusammensetzung unter Rühren mit 200-400 U/min und bei Umgebungstemperatur (18-25 °C) hindurchgeperlt wird für einen Zeitraum von 15 bis 100 Minuten, vorzugsweise 20 bis 40 Minuten, und dann dessen Einarbeitung in die entsprechende kosmetische oder dermatologische Formulierung erfolgt.

Während bei den bekannten Sauerstoff-Trägersystemen mit Phospholipiden oder zu den Phospholipiden zählenden Sphingomyelinen (Cerasome) nach einem anfänglichen Abfall der O₂-Konzentration nach 24 h von etwa 30-50 % in den Wochen danach ein kontinuierlicher Konzentrationsabfall bis auf das Gleichgewicht mit Luftsauerstoff nach 26 Wochen auftritt, halten die erfindungsgemäßen Transportsysteme nach dem 24 h-Abfall von 30-50 % in den Wochen danach die O₂-Konzentration deutlich länger und mit höheren Anteilen von 25-40 %. Nach 8 Wochen ergaben Messungen für das erfindungsgemäße Transportsystem O₂-Konzentrationen von 5-15 % gegenüber dem Anfangsgehalt, die dann weiterhin etwa konstant bleiben.

Das bedeutet, dass ein erhöhtes Sauerstoffangebot bei vergleichbarer Konzentration der Perfluorcarbone für einen längeren Zeitraum zur Verfügung steht und damit den Hautzellen zugeführt werden kann. Gerade im Oberflächenbereich der Haut bis etwa 0,4 mm Tiefe wird bekanntlich der größte Sauerstoffanteil nicht über das Blut, sondern über den Luftsauerstoff aufgenommen.

Weiterhin vorteilhaft kann das Sauerstoff-Trägersystem der Erfindung Tocopherol oder ein Tocopherolderivat mit einem Anteil von 0,01-1,5 Gew-% enthalten, bezogen auf das Gesamtgewicht des Trägersystems. Dadurch wird eine weitere Wirkungsverbesserung in Bezug auf die Stabilität des Systems in siliconölhaltigen kosmetischen Formulierungen erzielt. Geeignete Tocopherolderivate sind z.B. Tocopherylacetat, Tocopherylpalmitat, Tocopherylsuccinat, Tocopherylpropionat, Tocopheryloleat, Tocopheryllinolat oder Tocopherylsorbat, die auch im Gemisch untereinander oder mit Tocopherol eingesetzt werden können.

Das erfindungsgemäße Sauerstoff-Transportsystem kann überraschend in kosmetischen oder dermatologischen Formulierungen, insbesondere in solchen mit Siliconölen oder Siliconpolymeren, in Konzentrationen bis zu 40 Gew-% eingearbeitet sein, ohne die Stabilität der kosmetischen oder dermatologischen Formulierung zu beeinträchtigen.

Das flüssige Siliconpolymere in dem erfindungsgemäßen Trägersystem auf Ölbasis ist vorteilhaft ein cyclisches Siliconöl oder ein Gemisch von cyclischen mit anderen Siliconölen.

Das flüssige Siliconpolymere in einem Trägersystem auf Wasserbasis ist vorteilhaft eine wässrige Suspension von Siliconelastomeren in Kombination mit einem Methylpolysiloxan.

Besonders bevorzugt sind Organosiliconoxidpolymere, wie Methylpolysiloxane, wie z.B. BAR-SIL^{®} 2001 (von Barnet, Englewood Cliffs, USA) oder Fluorsilicone, die wenigstens eine Trifluoralkyl-gruppe im Monomeren enthalten, wie Gransil®, z.B. Gransil® FLD-55 (von Grant Ind., Inc, Elmwood Park, USA). Als Silicon-Elastomeremulsion ist bevorzugt Dimethicone/ Vinyldimethicone Crosspolymer (and) CI2-12 Pareth-12 mit einer Viskosität von etwa 5000 mPa.s.

Weitere geeignete Siliconpolymere sind Polydimethylcyclosiloxane (z.B. Cyclopentasiloxane), Gemische von Cyclopentasiloxane mit Cyclohexasiloxane (z.B. DC® 345), Gemische von Cyclopentasiloxane mit hochviskosem Dimethiconol (z.B. DC® 1501), Gemische von Polydimethylsiloxanen mit Viskositäten im Bereich von 0,65 - 60000 mm²s (z.B. DC® 200 Fluids).

Die Ölbasis des Trägersystems ist vorteilhaft ein pflanzliches Öl, ein Ester wie z.B. Dicaprylyl Carbonate (Cetiol CC), Isodecyl Neopentanoate (z.B. DUV VCI 10), Neopentyl Glycol Diheptanoate (z.B. Lexfeel® 7), Trimethylolpropane Tricaprylate/Tricaprate z.B (z.B. Lexfeel (®21) der ein Gemisch davon. Synthetische Öle sind besonders bevorzugt.

Der Sauerstoffträger ist bevorzugt ein perfluorierter Kohlenwasserstoff, insbesondere Perfluordecalin, F-Butyltetrahydrofuran, Perfluortributylamin, Perfluormethyl-cyclopentan, Perfluor-1,3-dimethylcyclohexan, Perfluorperhydrobenzyldecalin, Perfluorperhydrophenanthren, Perfluoroctylbromid, Bis-Fluor-(butyl)ethen oder C₆-C₉-Perfluoralkane.

Der Anteil des perfluorierten Kohlenwasserstoffs oder Kohlenwasserstoffgemisches liegt vorzugsweise im Bereich von 1,5 bis 6 Gew-%, bezogen auf das Gesamtgewicht des Trägersystems.

Der Anteil des Siliconpolymeren im Trägersystem liegt vorteilhaft bei 15 bis 35 Gel-%.

Das Trägersystem enthält weiterhin vorzugsweise wenigstens ein gelierendes Mittel mit einer Viskosität im Bereich von 120.000 Pa.s bis 300.000 Pa.s, insbesondere in einem Trägersystem auf Ölbasis.

Besonders bevorzugt sind solche Gele wie mineralölfreie Gele mit hydriertem Polyisobuten als Basis, wie Versagel^{®} (von Penreco, Dickinson, USA) oder organische, modifizierte Montmorillonitsorten wie Bentone Gel.

Bevorzugt ist auch der Einsatz von Verdickungsmitteln in dem Trägersystem.

Als Wasser-Verdickungsmittel werden vorteilhaft Xanthan Gum, Ammonium Acryloyldimethyl Laurate/VP Copolymer, Ammonium Acryloyldimethyl Laurate/Beheneth-25, Methylacrylate Copolymer (z.B. Aristoflex® AVC oder HMB), hochmolekulare Homo- und Copolymere von Acrylsäure und Polyalkenylpolyethern (z.B. Carbopol®-Verdicker) verwendet.

Als Öl-Verdickungsmittel sind verschiedene Bentongele geeignet, wie z.B. C12-15 Alkyl Benzoate(and)Stearalkonium Hectorite (and)Propylene Carbonate, Isodecane(and) Disteardimonium Hectorite(and)Propylene Carbonate, Cyclopentasiloxane(and)Disteardimonium Hectorite(and)SD Alcohol, die auch im Gemisch eingesetzt werden können.

Auch Verdickungsmittel wie hydriertes Polyisobuten (z.B. Versagel® ME 1600) mit einer Viskosität von etwa 143000 mPa.s (Brookfiled. Viskosimeter, 25 °C, Spindel T-C, 5 U/min) können verwendet werden.

Das erfindungsgemäße Sauerstoff-Trägersystem kann in beliebige kosmetische oder dermatologische Formulierungen eingearbeitet werden, z.B. kann eine solche topische Formulierung in Form eines Cremes, einer Lotion, eines Selbstbräunungsmittels, einer Sonnenschutzformulierung für vor, während und nach der Sonnenbehandlung, einer Maske, eines Gels, eines Sprays vorliegen.

In einer kosmetischen Formulierung kann der Anteil des Sauerstoff-Trägersystems 1 bis 25 Gew-% betragen, bezogen auf das Gesamtgewicht der Formulierung, vorzugsweise 6 bis 10 %.

In einer dermatologischen Formulierung kann der Anteil des Sauerstoff-Trägersystems 3 bis 40 Gew-%, vorzugsweise 6 bis 35 Gew-% betragen, bezogen auf das Gesamtgewicht der Formulierung.

Die kosmetischen oder dermatologischen Formulierungen, in die da erfindungsgemäße Sauerstoff-Trägersystem eingearbeitet werden kann, können übliche Hilfs-, Träger- und Wirkstoffe enthalten, z.B. Hilfs- und Trägerstoffe wie Wasser, Konservierungsmittel, Farbstoffe, Pigmente mit färbender Wirkung, Verdickungsmittel, Duftstoffe, einwertige und mehrwertige Alkohole, Ester, Elektrolyte, Gelbildner, polare und unpolare Öle, Polymere, Copolymere, Emulgatoren, Wachse, Stabilisatoren.

Besonders geeignet sind Formulierungen, die Anteile von Siliconölen > 5 % enthalten, vorzugsweise 6 - 70 %, insbesondere 6 - 35 %. In derartigen Formulierungen lassen sich die erfindungsgemäßen Sauerstoff-Transportsysteme ohne die Gefahr der Instabilität der Formel einarbeiten. Als Siliconöle in der Formulierung kommen alle gebräuchlichen Siliconöle in Frage.

Zu kosmetischen Wirkstoffen gehören z. B. anorganische und organische Lichtschutzmittel, Radikalfänger, Feuchthaltemittel, Vitamine, Enzyme, pflanzliche Wirkstoffe, Antioxidationsmittel, entzündungswidrige natürliche Wirkstoffe, Aufschlussprodukte von Hefen oder pflanzlichen Stoffen, hergestellt durch ein schonendes Ultraschall-Aufschlussverfahren gemäß WO 94/13783, Kaolin sowie mit SiO₂ modifiziertes Kaolin gemäß WO94/17588 und Dihydroxyaceton. Ein weiterer Wirkstoff für eine solche Formulierung mit dem erfindungsgemäßen Sauerstoffträgersystem ist eine spezielle Zubereitung mit hohem Radikalschutzfaktor mit einem Gehalt an einem durch Extraktion der Rinde von Quebracho blanco und nachfolgender enzymatischer Hydrolyse gewonnenem Produkt, das wenigstens 90 Gew-% Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen HydroGel oder Gemisch von Hydro-Gelen, und einem oder mehreren Phospholipiden, und Wasser. Diese spezielle Zubereitung kann weiterhin Superoxiddismutase und Cyclodextrin enthalten.

Die Erfindung betrifft weiterhin ein Verfahren zur Herstellung eines kosmetischen Sauerstoff-Trägersystems, das dadurch gekennzeichnet ist, dass man bei einer Temperatur im Bereich von 18 bis 26°C wenigstens in einen Teil der Öl- oder Wasserbasis wenigstens ein Teil eines flüssigen Siliconpolymeren unter Rühren mit 20 bis 100 U/Min einbringt, dazu unter Rühren mit 10 bis 80 U/Min einen flüssigen perfluorierten oder teilfluorierten Kohlenwasserstoff oder ein Kohlenwasserstoffgemisch einbringt und 3 bis 30 Minuten rührt, gegebenenfalls weitere Bestandteile oder restliche Anteile der genannten Bestandteile unter Rühren mit 40 bis 150 U/Min einbringt und das Gemisch für 20 bis 150 sec. mit maximal 3000 U/Min homogenisiert.

Vorteilhaft wird in dem Verfahren das Trägersystem mit gasförmigem Sauerstoff bis zu einem Partialdruck von 150 bis 950 mbar O₂ beladen.

Die Erfindung betrifft weiterhin die Verwendung eines kosmetischen Sauerstoff-Trägersystems, bestehend aus einem flüssigen perfluoriertem oder teilfluoriertem Kohlenwasserstoff oder Kohlenwasserstoffgemisch mit einem Anteil von 0,1-10 Gew-%, einem flüssigen Siliconpolymeren mit einem Anteil von 35-85 Gew-%, einer Öl- oder Wasserbasis mit einem Anteil von 5-25 Gew-% und gegebenenfalls einem Tocopherol oder Tocopherolderivat mit einem Anteil von 0,01-1,5 Gew-% , wobei alle Gewichtsanteile auf das Gesamtgewicht des Trägersystems bezogen sind in topischen Formulierungen, insbesondere in solchen mit Siliconölgehalten von 5-25 Gew-%, bezogen auf das Gesamtgewicht der topischen Formulierung.

Die topische Formulierung liegt vorteilhaft in Form eines Cremes, einer Lotion, eines Selbstbräunungsmittels, einer Sonnenschutzformulierung für vor, während und nach der Sonnenbehandlung, einer Maske, eines Gels, eines Sprays vor.

Die Erfindung soll nachstehend durch Beispiele näher erläutert werden. Alle Angaben erfolgen in Gewichtsprozent, sofern nichts anderes angegeben ist.

### Beispiel 1 Herstellung des Sauerstoff-Trägersystems (SiOx I) auf wässeriger Basis

| Zusammensetzung in Gew-%: | | |
|---|---|---|
| (A) | Dimethicone | 20 |
| (B) | Trifluoromethyl Cl-4 Alkyl Dimethicone | 35 |
| (C) | Perfluordecalin | 5 |
| (D) | Tocopherol | 0,4 |
| (E) | Dimethicone/Vinyldimethicone Crosspolymer | |
| | (and) CI2-14 Pareth-12 | 25 |
| (F) | Wasser | 14,6 |

Unter Rühren wird zu (A) bei Umgebungstemperatur die entsprechende Menge (B) langsam zugegeben und einige Minuten weitergerührt. Unter weiterem Rühren wird (C) zugesetzt und etwa 20 bis 30 Minuten weitergerührt. Danach wird (E) ebenfalls unter langsamem Rühren nacheinander zugegeben, dann (D) und schließlich (F). Das Gemisch wird mit etwa 2500 U/min für 50 Sek. homogenisiert. Alle Vorgänge erfolgten bei etwa 21-25 °C.

Danach wird gasförmiger Sauerstoff mit 400 mbar für 30 Minuten unter Rühren mit 280 U/min durch das Gemisch geperlt. Die erhaltenen Sauerstoffgehalte werden jeweils mit einem Oxi 3000 (WTW GmbH, Weilheim, Deutschland) gemessen.

### Beispiel 2 Herstellung des Sauerstoff-Trägersystems (SiOx II) auf Ölbasis

| Zusammensetzung in Gew-%: | | |
|---|---|---|
| (A) | Cyclopentasiloxane | 10 |
| (B) | Dicapryl Carbonate | 10 |
| (C) | Perfluordecalin | 2 |
| (D) | Tocopherol | 0,5 |
| (E) | Hydrogenated Polyisobutene (and) Ethylene/Propylene/Styrene Copolymer(and) Butylene/-Ethylene/-Styrene Copolymer | |
| | (Versagel® ME1600) | 40 |
| (F) | Bentone Gel | 37,5 |

Unter Rühren mit 400 bis 500 U/min wird zu (A) die entsprechende Menge (B) langsam zugegeben und einige Minuten weitergerührt. Mit 900 bis 1000 U/min wird (C) zugesetzt und etwa 20 bis 30 Minuten weitergerührt. Danach wird (D) hinzugegeben, dann (E) und schließlich (F). Das Gemisch wird mit etwa 2700 bis 2800 U/min für 40 Sek. homogenisiert. Alle Vorgänge erfolgten bei etwa 20-24 °C.

Danach wird gasförmiger Sauerstoff mit 450 mbar für 35 Minuten unter Rühren mit 300 U/min durch das Gemisch geperlt.

### Beispiel 3 Körperschaum (Moussé) I

| | |
|---|---|
| Wasser | bis 100% |
| Squalane | 0,01 |
| Butylengylcol | 2,0 |
| Glycerin | 2,0 |
| PPG-5 Ceteth-20 | 1,0 |
| Decylglucosid | 3,0 |
| SiOx I von Beispiel 1; 160 mbar O₂ | 6,0 |

Die Bestandteile wurden in der angegebenen Reihenfolge bei Raumtemperatur vermischt and dann in eine Pumpflasche mit einer speziellen Pumpe für Mousse gegeben.

### Beispiel 4 Körperschaum (Moussé) II

Die gleichen Bestandteile wie im Beispiel 3 und zusätzlich 6 % Siliconöl wurden in gleicher Weise zu einem Körperschaum verarbeitet.

### Beispiel 5 Selbstbräunungsmittel I O/W

| **Phase A** | |
|---|---|
| Wasser | bis 100% |
| Dihydroxyaceton | 5,0 |
| Glycerin | 2,0 |

| **Phase B** | |
|---|---|
| Dimethicone | 6,0 |
| Dimethicone/Vinyldimethicone Crosspolymer | |
| (and) CI2-14 Pareth-12 | 25 |
| Cyclopentasiloxan Dimethiconol | 2,0 |
| Octyldodecyl Stearoyl Stearate | 2,5 |

| **Phase C** | |
|---|---|
| Konservierungsmittel | 0,5 |
| Parfüm | 0,5 |

| **Phase D** | |
|---|---|
| SiOx II von Beispiel 2; 210 mbar O₂ | 10 |

### Beispiel 6 Selbstbräunungsmittel II (Vergleichsbeispiel)

| Phase A | |
|---|---|
| Wasser | bis 100 |
| Dihydroxyaceton | 5,0 |
| Glycerin | 2,0 |

| **Phase B** | |
|---|---|
| Dimethicone | 6,0 |
| Dimethicone/Vinyldimethicone Crosspolymer | |
| (and) CI2-14 Pareth-12 | 5 |
| Cetiole CC | 20 |
| Octyldodecyl Stearoyl Stearate | 2,5 |

| **Phase C** | |
|---|---|
| Konservierungsmittel | 0,5 |
| Parfüm | 0,5 |
| Asymmetrische lamellare Aggregate gemäß | |
| WO94/0098 | 5 |

Bei dem Versuch, den Gehalt der zuletzt genannten Aggregate als Sauerstoffträger (180 mbar O₂) über 5 % zu erhöhen, wurde die Formel instabil und es trat Phasentrennung auf.

### Beispiel 7 Körpergel

| | |
|---|---|
| Wasser | bis 100 |
| Glycerin | 5 |
| Dimethicone | 15 |
| Propylenglycol | 10 |
| Carbomer | 2 |
| Triethanolamin | 2 |
| Konservierungsmittel | 0,5 |
| Parfümöl | 0,2 |
| SiOx I von Beispiel 1; 254 mbar O₂ | 20 |

Die Bestandteile wurden nacheinander zusammengegeben und homogen verrührt.

### Beispiel 8 Haut-Fluidserum

| | |
|---|---|
| Wasser | bis 100 |
| Glycerin | 3 |
| Propylenglycol | 5 |
| Carbomer | 1 |
| Silicone | 10 |
| Triethanolamin | 1 |
| Konservierungsmittel | 0,5 |
| Parfümöl | 0,2 |
| SiOx I von Beispiel 2; 215 mbar O₂ | 15 |

Die Bestandteile wurden nacheinander zusammengegeben und homogen verrührt.

## Patentansprüche

1. Kosmetische oder dermatologische Formulierung mit einem Sauerstoff-Trägersystem, **dadurch gekennzeichnet, dass** das Sauerstoff-Trägersystem aus einem flüssigen perfluorierten oder teilfluorierten Kohlenwasserstoff oder Kohlenwasserstoffgemisch mit einem Anteil von 0,1-10 Gew-%, einem flüssigen Siliconpolymeren oder Siliconpolymerengemisch mit einem Anteil von 10-85 Gew-%, einer Öl- oder Wasserbasis mit einem Anteil von 5-25 Gew-% besteht, wobei alle Gewichtsanteile auf das Gesamtgewicht des Trägersystems bezogen sind, und das Sauerstoff-Trägersystem in der Formulierung in einem Anteil von 1-40 Gew-% vorliegt, bezogen auf das Gesamtgewicht der Formulierung und wobei das Trägersystem mit gasförmigem Sauerstoff bis zu einem Partialdruck von 150-950 mbar O₂ beladen ist.

2. Formulierung nach Anspruch 1,
**dadurch gekennzeichnet, dass** das Trägersystem mit einem Sauerstoffgehalt vier Wochen nach der Beladung in Höhe von 25-40 Vol-% des Anfangs-Sauerstoffgehaltes beladen ist.

3. Formulierung nach Anspruch 1,
**dadurch gekennzeichnet, dass** das Trägersystem Tocopherol oder ein Tocopherolderivat mit
einem Anteil von 0,01-1,5 Gew-% enthält.

4. Formulierung nach Anspruch 1,
**dadurch gekennzeichnet, dass** der Anteil des flüssigen Siliconpolymeren im Bereich von 15 - 35 Gew-% liegt.

5. Formulierung nach Anspruch 1,
**dadurch gekennzeichnet, dass** das Trägersystem wenigstens ein gelierendes oder verdickendes Mittel oder ein Gemisch davon enthält.

6. Formulierung nach Anspruch 1,
**dadurch gekennzeichnet, dass** die Ölbasis des Trägersystems ein pflanzliches Öl, ein Ester oder ein Gemisch davon ist.

7. Formulierung nach Anspruch 1,
**dadurch gekennzeichnet, dass** der Sauerstoffträger Perfluordecalin ist.

8. Formulierung nach Anspruch 1,
**dadurch gekennzeichnet, dass** der Anteil des perfluorierten Kohlenwasserstoffs oder Kohlenwasserstoffgemisches im Bereich von 1,5 bis 6 Gew-% liegt.

9. Formulierung nach Anspruch 1,
**dadurch gekennzeichnet, dass** das Sauerstoff-Trägersystem in einem Anteil von 6 bis 10 % vorliegt.

10. Formulierung nach Anspruch 1,
**dadurch gekennzeichnet, dass** sie als kosmetische Formulierung ein Siliconöl in einem Anteil von 3 - 70 % enthält, bezogen auf das Gesamtgewicht der Formulierung, vorzugsweise einen Anteil von 6 - 35 %.

11. Formulierung nach Anspruch 1,
**dadurch gekennzeichnet, dass** das Sauerstoff-Trägersystem in einer Formulierung in einem Anteil von 3 bis 40 Gew-%, vorzugsweise 6 bis 35 Gew-% vorliegt, bezogen auf das Gesamtgewicht der Formulierung.

12. Verfahren zur Herstellung einer kosmetischen oder dermatologischen Formulierung mit einem kosmetischen Sauerstoff-Trägersystem nach Anspruch 1,
**dadurch gekennzeichnet, dass** man bei einer Temperatur im Bereich von 18 bis 26°C wenigstens in einen Teil der Öl- oder Wasserbasis wenigstens einen Teil eines flüssigen Siliconpolymeren unter Rühren mit 20 bis 100 U/Min einbringt, dazu unter Rühren mit 10 bis 80 U/Min einen flüssigen perfluorierten oder teilfluorierten Kohlenwasserstoff oder ein Kohlenwasserstoffgemisch einbringt und 3 bis 30 Minuten rührt, gegebenenfalls weitere Bestandteile für das Trägersystem oder restliche Anteile der genannten Bestandteile unter Rühren mit 40 bis 150 U/Min einbringt und das Gemisch für 20 bis 150 sec. mit maximal 3000 U/Min 10 bis 40 Minuten homogenisiert, das Trägersystem mit gasförmigem Sauerstoff bis zu einem Partialdruck von 150-950 mbar O₂ belädt und das Trägersystem mit Bestandteilen der kosmetischen oder dermatologischen Formulierung vermischt.

13. Verfahren nach Anspruch 12,
**dadurch gekennzeichnet, dass** das Trägersystem mit gasförmigem Sauerstoff mit einem Partialdruck von 180 bis 600 mbar behandelt wird.

14. Verwendung einer kosmetischen Formulierung mit einem Sauerstoff-Trägersystem, bestehend aus einem flüssigen perfluoriertem oder teilfluoriertem Kohlenwasserstoff oder Kohlenwasserstoffgemisch mit einem Anteil von 0,1-10 Gew-%,
einem flüssigen Siliconpolymeren mit einem Anteil von 35-85 Gew-%,
einer Öl- oder Wasserbasis mit einem Anteil von 5-25 Gew-%,
wobei alle Gewichtsanteile auf das Gesamtgewicht des Trägersystems bezogen sind, wobei das Trägersystem mit gasförmigem Sauerstoff bis zu einem Partialdruck von 150-950 mbar O₂ beladen ist, in topischen Formulierungen mit Siliconölgehalten von 5-25 Gew-%, bezogen auf das Gesamtgewicht der topischen Formulierung.

15. Verwendung nach Anspruch 14, **dadurch gekennzeichnet, dass** die topische Formulierung in Form eines Cremes, einer Lotion, eines Selbstbräunungsmittels, einer Sonnenschutzformulierung für vor, während und nach der Sonnenbehandlung, einer Maske, eines Gels, eines Sprays vorliegt.

## Claims

1. Cosmetic or dermatologic formulation with an oxygen carrier system which comprises a liquid perfluorinated or partially fluorinated hydrocarbon or hydrocarbon mixture with a moiety of 0.1 - 10% by weight, a liquid silicone polymer or silicone polymer mixture with a moiety of 10 - 85% by weight and an oil or water base with a moiety of 5 - 25% by weight, with all weight percentages being related to the total weight of the carrier system and the oxygen carrier system is present in the formulation with a moiety of 1 - 40% by weight, related to the overall weight of the formulation, and the carrier system being loaded with gaseous oxygen up to a partial pressure of 150 - 950 mbar O₂.

2. Formulation according to Claim 1, wherein the carrier system is loaded four weeks after the loading with an oxygen content of 25 - 40% by volume of the initial oxygen content.

3. Formulation according to Claim 1, wherein the carrier system contains tocopherol or a tocopherol derivative with a moiety of 0.01 - 1.5% by weight.

4. Formulation according to Claim 1, wherein the moiety of the liquid silicone polymer ranges from 15 - 35% by weight.

5. Formulation according to Claim 1, wherein the carrier system contains at least a gelling or thickening agent or a mixture thereof.

6. Formulation according to Claim 1, wherein the oil base of the carrier system is a vegetable oil, an ester or mixture thereof.

7. Formulation according to Claim 1, wherein the oxygen carrier is perfluorodecaline.

8. Formulation according to Claim 1, wherein the moiety of the perfluorinated hydrocarbon or hydrocarbon mixture ranges from 1.5 - 6% by weight.

9. Formulation according to Claim 1, wherein the oxygen carrier system is present in a moiety of 6 to 10%.

10. Formulation according to Claim 1, wherein it contains as cosmetic formulation a silicone oil with a moiety of 3 - 70%, related to the overall weight of the formulation, preferably with a moiety of 6 - 35%.

11. Formulation according to Claim 1, wherein the oxygen carrier system in a formulation is present with a moiety of 3 to 40% by weight, preferably 6 to 35% by weight, related to the overall weight of the formulation.

12. Procedure for the preparation of cosmetic or dermatologic formulation with a cosmetic oxygen carrier system according to Claim 1, wherein at least one part of a liquid silicone polymer is incorporated while stirring at 20 to 100 r.p.m. into at least one part of the oil or water base at a temperature ranging from 18 to 26 °C, adding in addition while stirring at 10 to 80 r.p.m. a liquid perfluorinated or partially perfluorinated hydrocarbon or a hydrocarbon mixture and stirring it for 3 to 30 minutes, adding, optionally, further constituents for the carrier system or remaining moieties of the mentioned constituents while stirring at 40 to 150 r.p.m. and homogenising the mixture for 20 to 150 seconds at maximally 3,000 r.p.m. 10 to 40 minutes, loading the carrier system with gaseous oxygen up to a partial pressure of 150 - 950 mbar O₂ and mixing the carrier system with constitutents of the cosmetic or dermatologic formulation.

13. Procedure according to Claim 12, wherein the carrier system is treated with gaseous oxygen with a partial pressure of 180 to 600 mbar.

14. Use of a cosmetic formulation with an oxygen carrier system, comprising a liquid perfluorinated or partially perfluorinated hydrocarbon or hydrocarbon mixture with a moiety of 0.1 - 10% by weight, a liquid silicone polymer with a moiety of 35 - 85% by weight, an oil or water base with a moiety of 5 - 25% by weight, all weight percentages being related to the overall weight of the carrier system, in topical formulations with silicone oil contents of 5 - 25% by weight, related to the overall weight of the topical formulation, and the carrier system being loaded with gaseous oxygen up to a partial pressure of 150 - 950 mbar O₂.

15. Use according to Claim 14 wherein the topical formulation has the form of a cream, a lotion, a self-tanning agent, a sun protection formulation for use before, during and after the exposure to sun, of a mask, a gel, a spray.

## Revendications

1. Formulation cosmétique ou dermatologique comportant un excipient à base d'oxygène, **caractérisée en ce que** l'excipient à base d'oxygène se compose d'un carbure d'hydrogène liquide perfluoré ou partiellement perfluoré ou d'un mélange à base de carbure d'hydrogène en une proportion de 0,1-10 % en poids, d'un polymère silicone liquide ou d'un mélange à base de polymère silicone en une proportion de 10-85 % en poids, d'une base huileuse ou d'une base aqueuse en une proportion de 5-25 % en poids, toutes les proportions en poids étant rapportées au poids total de l'excipient et l'excipient à base d'oxygène étant présent dans la formulation en une proportion de 1-40 % en poids, rapporté au poids total de la formulation, et l'excipient comportant de l'oxygène sous forme gazeuse étant chargé jusqu'à une pression partielle de 150-950 mbar d'O₂.

2. Formulation selon la revendication 1,
**caractérisée en ce que** l'excipient comportant une teneur en oxygène est chargé à hauteur de 25-40 % en volume de la teneur en oxygène initiale quatre semaines après le chargement.

3. Formulation selon la revendication 1,
**caractérisée en ce que** l'excipient contient du tocophérol ou un dérivé du tocophérol en
une proportion de 0,01-1,5 % en poids.

4. Formulation selon la revendication 1,
**caractérisée en ce que** la proportion du polymère silicone liquide se situe dans la plage allant de 15 à 35 % en poids.

5. Formulation selon la revendication 1,
**caractérisée en ce que** l'excipient contient au moins un agent gélifiant ou épaississant ou un mélange de ces derniers.

6. Formulation selon la revendication 1,
**caractérisée en ce que** la base huileuse de l'excipient est une huile végétale, un ester ou un mélange de ces derniers.

7. Formulation selon la revendication 1,
**caractérisée en ce que** le porteur d'oxygène est une perfluorodécaline.

8. Formulation selon la revendication 1,
**caractérisée en ce que** la proportion du carbure d'hydrogène perfluoré ou du mélange à base de carbure d'hydrogène se situe dans la plage allant de 1,5 à 6 % en poids.

9. Formulation selon la revendication 1,
**caractérisée en ce que** l'excipient à base d'oxygène est présent en une proportion allant de 6 à 10 % en poids.

10. Formulation selon la revendication 1,
**caractérisée en ce qu'**elle contient en tant que formulation cosmétique une huile de silicone en une proportion de 3-70 %, rapportée au poids total de la formulation, de préférence en une proportion de 6-35 %.

11. Formulation selon la revendication 1,
**caractérisée en ce que** l'excipient à base d'oxygène est présent dans une formulation en une proportion allant de 3 à 40 % en poids, de préférence allant de 6 à 35 % en poids, rapportée au poids total de la formulation.

12. Procédé de fabrication d'une formulation cosmétique ou dermatologique comportant un excipient cosmétique à base d'oxygène selon la revendication 1,
**caractérisé en ce que** l'on introduit en agitant à une vitesse de 20 à 100 t/min. au moins une partie d'un polymère silicone liquide, à une température se situant dans la plage allant de 18 à 26 °C, au moins dans une partie de la base huileuse ou aqueuse, **en ce que** l'on y ajoute en agitant à une vitesse de 10 à 80 t/min. un carbure d'hydrogène liquide perfluoré ou partiellement perfluoré ou un mélange à base de carbure d'hydrogène, **en ce que** l'on introduit le mélange pendant une durée allant de 3 à 30 minutes, **en ce que** l'on introduit en agitant à une vitesse de 40 à 150 t/min. le cas échéant d'autres composants pour l'excipient ou des proportions résiduelles desdits composants, et **en ce que** l'on homogénéise le mélange pour 20 à 150 secondes avec une vitesse maximale de 3000 t/min. pendant 10 à 40 minutes, **en ce que** l'excipient comportant de l'oxygène sous forme gazeuse est chargé jusqu'à une pression partielle de 150-950 mbar d'O₂, et **en ce que** l'on mélange l'excipient avec des composants de la formulation cosmétique ou dermatologique.

13. Procédé selon la revendication 12,
**caractérisé en ce que** l'excipient comportant de l'oxygène sous forme gazeuse est chargé avec une pression partielle allant de 180 à 600 mbar.

14. Utilisation d'une formulation cosmétique comportant un excipient à base d'oxygène dans des formulations topiques comportant des teneurs en huile de silicone de 5-25 % en poids, rapportées au poids total de la formulation topique, ledit excipient se composant d'un carbure d'hydrogène liquide perfluoré ou partiellement perfluoré ou d'un mélange à base de carbure d'hydrogène en une proportion de 0,1-10 % en poids,
d'un polymère silicone liquide en une proportion de 35-85 % en poids,
d'une base huileuse ou d'une base aqueuse en une proportion de 5-25 % en poids, toutes les proportions en poids étant rapportées au poids total de l'excipient, l'excipient comportant un oxygène sous forme gazeuse étant chargé jusqu'à une pression partielle de 150-950 mbar d'O₂.

15. Utilisation selon la revendication 14, **caractérisée en ce que** la formulation topique est présente sous la forme d'une crème, d'une lotion, d'un agent autobronzant, d'une formulation de protection solaire pour une protection avant, pendant et après exposition, d'un masque, d'un gel, d'un spray.
